# EUROPEAN PATENT APPLICATION

(11) **EP 4 664 476 A1**
(43) Date of publication of application: **17.12.2025**
(21) Application number: 24181901.0
(22) Date of filing: 13.06.2024
(51) Int. Cl.: G16H 40/63, A61B 5/00, A61M 21/02

(54) **SLEEP MANAGEMENT DURING MEDICAL EXAMINATIONS**

(71) Applicant: Koninklijke Philips N.V., 5656 AG Eindhoven (NL)
(72) Inventor: NAUTS, Sanne, Eindhoven (NL); HEUVELINK, Annerieke, Eindhoven (NL); HELLE, Michael Günter, 5656AG Eindhoven (NL); KATEMANN, Christoph, Eindhoven (NL); FRERKING, Lena Christina, Eindhoven (NL); VAN EE, Raymond, Eindhoven (NL); WIEMKER, Rafael, Eindhoven (NL)
(74) Representative: Philips Intellectual Property & Standards

(57) **Abstract**

The present disclosure relates to a system and method for managing a subject's sleep state during a medical examination. In particular, the present disclosure may provide systems and methods for monitoring a subject's sleep state and adjusting the operation parameters of a subject guidance and entertainment system based on the subject's current sleep state and a target sleep state. Disclosed embodiments thus enable the automatic adjustment of the subject's environment to either facilitate sleep or wakefulness. This may be particularly beneficial during lengthy medical procedures, such as MRI scans, where subjects often fall asleep.

## Description

### FIELD OF INVENTION

The present invention pertains to the field of medical technology, specifically to systems and methods for managing a subject's sleep/wake state during medical examinations such as MRI scans.

### BACKGROUND OF THE INVENTION

Medical procedures, particularly those involving radiology, often require subjects to remain still for extended periods of time. This is particularly true for procedures such as Magnetic Resonance Imaging (MRI) scans, which can take up to an hour or more to complete. During these procedures, subjects are asked to remain as motionless as possible to ensure the accuracy of the images being captured.

In many cases, due to the length of the procedure and the comfortable, warm environment, the subject may fall asleep. This is especially common among subjects who have taken medication that induces drowsiness, such as anxiolytics, or among those predisposed to sleep. However, even those who are simply tired or comfortable may also fall asleep during the procedure.

While a sleeping subject may not pose a problem in some cases, there are instances where subject movement during sleep can negatively impact the quality of the images captured during the procedure. For example, if a subject moves a lot during their sleep, this can cause motion artifacts in the MRI images. Additionally, subjects who doze off and then suddenly wake up can also cause motion that negatively affects the image quality.

Furthermore, there are instances where the subject is expected to be awake during part of the procedure to comply with instructions. For example, subjects may be asked to hold their breath during specific parts of the scan or to complete tasks during functional MRI (fMRI) scans. In these cases, waking up the subject can be a cumbersome task for the examiner, and the sudden awakening may startle the subject, potentially causing further motion and requiring new reference scans or planning.

Accordingly, there exists a need for an improved means for managing a subject sleep/wake state during a medical examination/procedure.

### SUMMARY OF INVENTION

The invention is defined by the claims.

According to an aspect of the present disclosure, a computer-implemented method is provided for managing a sleep state of a subject during a medical examination. The method includes obtaining a target sleep status of the subject, detecting a current sleep status of the subject, and adapting one or more operation parameters of a subject guidance and entertainment system based on the current sleep status and the target sleep status.

Proposed concepts thus provide a method for managing a subject's sleep state during a medical examination. In particular, the present disclosure may provide systems and methods for monitoring a subject's sleep state and adjusting the operation parameters of a subject guidance and entertainment system based on the subject's current sleep state and a target sleep state. Disclosed embodiments thus enable the automatic adjustment of the subject's environment to either facilitate sleep or wakefulness. This may be particularly beneficial during lengthy medical procedures, such as MRI scans, where subjects often fall asleep.

In particular, embodiments aim to provide concepts for management a subject's sleep/wake state during lengthy medical procedures such as MRI scans. The disclosed concepts leverage the idea of monitoring the current sleep/wake state of a subject, for example using physiological parameters such as heart rate and breathing rate. Then, adjustments are made to the subject entertainment and guidance system, thereby potentially altering lighting, sound in the surrounding area of the subject, and/or content presented to the subject to either facilitate sleep or wakefulness, depending on the requirements of the medical examination and/or preferences of the subject and examiner. Furthermore, ventilation, room temperature, and operating parameters of an apparatus of the medical examination can be altered to further facilitate sleep or wakefulness.

The system leverages the idea that a subject's sleep/wake state can have a direct impact on the quality of the medical procedure. For instance, a subject who is asleep and calm may not induce motion artifacts in the MRI images, thereby resulting in high-quality scans. On the other hand, a subject who is asleep but restless or who frequently wakes up and falls back asleep can cause motion that negatively affects the image quality. In addition, some subjects may prefer to be in a certain wake/sleep state, and certain parts of the medical examination may require the subject to be in a particular wake/sleep state. Therefore, by monitoring and adjusting the subject's sleep/wake state, the system can help to ensure the quality of the medical procedure.

The advantages provided by the system include improved subject comfort and experience during medical procedures, as well as improved workflow of the medical procedure. By automatically adjusting the subject entertainment and guidance based on the subject's sleep/wake state, the system can help to prevent the subject from being startled awake, which can cause motion and disrupt the procedure. Furthermore, by facilitating sleep or wakefulness as appropriate, the method may automatically ensure that the subject is in the desired state of consciousness for the procedure, whether that be awake or asleep. This can help to ensure the quality of the procedure and improve the subject's experience, as well as reducing the burden on medical staff.

In another aspect of the present disclosure, the target sleep status of the subject may be based on a timetable of actions that are to be performed by the subject in relation to the medical examination. In this case, obtaining the target sleep status may comprise obtaining the timetable of actions and processing the medical examination timetable to determine the target sleep status of the subject. This allows for the system to anticipate when the subject will be expected to be awake or asleep for performing the medical examination, and adjust the environment accordingly.

In yet another aspect of the present disclosure, the target sleep status may be based on a user preference or a clinician preference. In this case, obtaining the target sleep status may comprise obtaining the user preference and/or the clinician preference, and determining the target sleep status of the subject based on the user preference and/or the clinician preference This allows for the system to be tailored to the specific requirements or preferences of the subject or the medical staff, providing a more personalized experience.

In a further aspect of the present disclosure, the method may include detecting a prompted sleep status of the subject responsive to the adaptation of the one or more operation parameters of the subject guidance and entertainment system, and further adapting one or more operation parameters of the subject guidance and entertainment system based on the prompted sleep status and the target sleep status. This allows for the system to respond dynamically to changes in the subject's sleep status, ensuring that the subject responds to the changes to the guidance and entertainment system in the expected manner.

In another aspect of the present disclosure, the method may include predicting a future sleep status of the subject based on the current sleep status of the subject, and optionally a previous sleep status of the subject, and wherein adapting the one or more operation parameters is further based on the predicted future sleep status. This predictive capability allows for the system to anticipate changes in the subject's sleep status and adjust the environment in advance, providing a smoother and more comfortable experience for the subject.

In some embodiments of the invention, adapting the one or more operation parameters of a subject guidance and entertainment system may comprise gradually changing the operation parameters from a current set of operation parameters to a target set of operation parameters, the target operation parameter based on the current sleep status and the target sleep status. By gradually changing the operation parameters, abrupt changes that could startle or disturb the subject may be avoided, thereby maintaining a more consistent and controlled examination environment.

In some cases, detecting the current sleep status of the subject may comprise obtaining at least one of a physiological signal of the subject during the medical examination, images of the subject during the medical examination, or behavior data describing actions performed by the subject during the medical examination. The current sleep status of the subject may then be determined based on the physiological signal, images, and/or behavior data of the subject. By utilizing a combination of physiological signals, images, and behavior data, the method may achieve a more accurate and reliable assessment of the subject's sleep state. Furthermore, by basing the determination of the current sleep status on a variety of data types, the method can potentially reduce the likelihood of false readings and improve the overall effectiveness of the sleep state management during the medical procedure.

In some embodiments, the subject guidance and entertainment system may comprise a display for observation by the subject during the medical examination. In this case, adapting the one or more operation parameters of the subject guidance and entertainment system may comprise adjusting a brightness level of the display, adjusting a color setting of the display, pausing or playing content presented by the display, or adjusting content presented by the display. Thus, the system can create an environment that is conducive to either promoting relaxation and sleep or stimulating wakefulness, as per the requirements of the medical examination.

In addition, the subject guidance and entertainment system may comprise a speaker for producing sound audible to the subject during the medical examination. In this case, adapting the one or more operation parameters of the subject guidance and entertainment system may comprise adjusting a volume of sound produced by the speaker, adjusting parameters of the sound produced by the speaker, pausing or playing sound produced by the speaker, or adjusting content of the sound produced by the speaker. This can lead to increased subject comfort, reduced likelihood of involuntary movement due to sudden sounds, and improved overall quality of the medical examination results.

In yet another aspect of the present disclosure, the method may include adapting one or more operation parameters of an apparatus for performing the medical examination based on the current sleep status and the target sleep status. This allows for the system to not just adjust the entertainment and guidance system, but also the operation of the medical equipment itself, potentially improving the quality of the medical examination.

In a further aspect of the present disclosure, the method may include generating a monitoring signal for indicating the sleep status of the subject to an examiner of the medical examination. This provides valuable feedback to the medical staff, allowing them to monitor the subject's sleep status and adjust their actions accordingly.

In another aspect of the present disclosure, the target sleep status may describe a target sleep stage of the subject, and the current sleep status of the subject may describe a sleep stage that the subject is currently experiencing. The sleep stage of the subject may include at least one of wake, N1, N2, N3, and REM. This allows for the system to distinguish between different stages of sleep (rather than just sleep and wake), and adjust the environment and the operation of the medical equipment accordingly.

In yet another aspect of the present disclosure, adapting one or more operation parameters of a subject guidance and entertainment system may be further based on one or more user characteristics. This allows for the system to be personalized to the specific traits and preferences of the subject, providing a more comfortable and enjoyable experience.

According to additional embodiments in accordance with the invention, there is provided a computer program code means adapted, when said computer program is run on a computer, to implement the method for managing a sleep state of a subject during a medical examination as described herein.

According to further embodiments in accordance with the invention, there is provided a system for managing a sleep state of a subject during a medical examination. The system includes an interface configured to obtain a target sleep status of the subject, a monitoring arrangement configured to detect a current sleep status of the subject, and a controller configured to adapt one or more operation parameters of a subject guidance and entertainment system based on the current sleep status and the target sleep status. This system provides a comprehensive solution for managing the sleep state of a subject during a medical examination, potentially improving the subject's comfort and the quality of the medical examination.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a flowchart of a method for managing a subject's sleep status during a medical procedure, according to aspects of the present disclosure;
Fig. 2 depicts a block diagram of a system for facilitating a subject's sleep/wake state during a medical examination, according to aspects of the present disclosure; and
Fig. 3 is a simplified block diagram of a computer within which one or more parts of an embodiment may be employed.

### DETAILED DESCRIPTION OF EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure relates to a system, method, and device for managing a subject's (i.e., a patient's) sleep state during a medical examination. In particular, the present disclosure may provide systems and methods for monitoring a subject's sleep state and adjusting the operation parameters of a subject guidance and entertainment system based on the subject's current sleep state and a target sleep state. This may be particularly beneficial during lengthy medical procedures, such as MRI scans, where subjects often fall asleep.

In many cases, a subject who sleeps calmly does not provide problems for the examination. Subjects who sleep calmly may not induce motion artefacts, may be easy to handle for staff, and generally have a positive experience (the scan is over before they know it). However, if subjects move a lot during their sleep, this can pose a problem for the image quality of the scan. Indeed, it may not be pleasant for a subject to doze off, suddenly wake up, and fall asleep again, and it may cause motion that negatively affects the image quality of the scan. Moreover, in some cases, subjects may need to be awake for part of the exam to comply with instructions from staff (i.e., an examiner, caregiver, or medical professional). In these cases, waking up subjects can be cumbersome for staff, and suddenly waking up may startle subjects, which can result in motion (e.g., displacement of the body part being scanned, potentially requiring new reference scans/planning).

Subject entertainment and guidance systems may positively affect subject experience and the workflow of the medical examination. They may ensure that the subject has a comfortable and engaging time by appropriately controlling the ambient environment (e.g., dynamic lighting, ambient temperature, etc.) and providing means of enjoyment (e.g., music, video, etc.), whilst issuing instructions to the subject throughout the medical examination. It has been noticed, however, that if a subject falls asleep while background entertainment/guidance is playing, the entertainment or guidance can inadvertently wake up the subject. For example, a subject may fall asleep but wake up because of an instruction from the system, because of the music played by the system, or because of a change in a visual aspect of the system (e.g., screen brightness or brightness of ambient lighting increasing as a result of a change in the movie/visuals of subject entertainment). If a subject is frequently changing sleep state due to the subject entertainment and guidance system, this may be suboptimal in terms of subject experience, staff experience, and image quality.

Thus, the invention proposes to reverse this potential disadvantage of subject entertainment and guidance systems into an advantage. Specifically, it is proposed to alter operation parameters of the subject entertainment and guidance system (e.g., brightness, volume, content type, etc.) based on knowledge of the subject's sleep/wake state, and a target sleep/wake state. Accordingly, the subject entertainment and guidance system may be adapted to facilitate and/or induce sleep when appropriate, whilst also facilitating and/or inducing wakefulness of the subject when appropriate. Thus, the subject may not inadvertently switch between various sleep states, but may be managed through various sleep states throughout the medical examination.

For example, if a subject needs to be awake for specific parts of the scan (or is liable to moving whilst sleeping), they may be woken up gradually to avoid startling by gradually altering operating parameters of the subject entertainment and guidance system (e.g., volume increased, brightness increased). Alternatively, when it is detected that the subject is beginning to fall asleep, operation parameters of the subject entertainment and guidance system may be altered to be more engaging (e.g., by providing alternative content, increasing brightness, or increasing volume) to decrease a likelihood of the subject falling asleep. Equally, if the subject may be asleep for part of the scan, they may be eased into a sleep (and sleep may be maintained) by gradually altering operating parameters of the subject entertainment and guidance system to be less stimulating. Of course, if it is detected that the subject is already asleep, then operating parameters of the subject entertainment and guidance system may be altered to pause content, so as to avoid waking the subject.

In one aspect, content (e.g., movies, audiobooks, music, etc.) provided by the subject entertainment and guidance system is paused when it is detected that the subject is asleep. Said content may then be resumed once it is detected that the subject has awoken. For example, if the subject starts falling asleep at 08:51 of a movie, then when the subject wakes up the movie will be resumed at 8:51 so they do not miss any of the movie.

Various embodiments of the invention thus provide means for monitoring the wake/sleep state (and various in-between states thereof) of a subject. If the subject varies from a target sleep state, operation of a subject entertainment and guidance system, as well as lighting, ventilation and room temperature, can be adjusted to manage the subject toward the target sleep state. That is, if the subject needs to be awake, they may be prompted toward wakefulness or wakefulness may be maintained. If the subject does not need to be awake, then settings may be adapted to avoid interrupting sleep.

Embodiments may consist of the following key elements:
(i) Sleep monitoring. Embodiments may provide a means for monitoring a subject's wake/sleep status. This can be done using different sensors, such as camera-based sensors, ECG, PPG, respiratory bellow, respiration rate, radar, etc. Indeed, if a subject falls asleep, this will lead to changes in heart rate, heart rate variability (HRV), breathing rate, facial appearance, and posture. Moreover, behavior (e.g. not responding to communication, or not following guidance) may be indicative of the subject's wake/sleep state. Another, indirect, way of tracking or fading attention is when the performance on a repetitive task is dropping, for instance as measured in reaction time or percentage correct answers for a task presented on screen.

Other means for assessing the current sleep state may also be employed such as using data from an MRI, EEG, pressure monitoring mattress, or by measuring the temperature or muscle tone of the subject. In some situations, it is also possible to indirectly detect the current sleep state of the subject. That is, actions and/or responses of the subject may be analyzed to determine a current sleep state, and a trajectory of the current sleep state. For example, a response speed and/or a response accuracy to certain questions may indicate the sleep state of the subject (if the subject has a slowing response speed, or increasing inaccuracy of response, this may mean that the subject is approaching sleep).

Thus, as will be appreciated, the invention may employ any suitable means for tracking the sleep state of the subject.
(ii) Facilitating sleep. When it is allowable for the subject to be asleep based on, for example, a medical examination timetable, subject and clinician preferences, operation parameters of the subject entertainment and guidance system may be automatically adjusted to facilitate sleep (in line with the detected/current subject sleep status).

When it is detected that the subject is asleep, or falling asleep the following may occur:
Content presented by the subject entertainment and guidance system (e.g., movies/themes) may be paused, brightness of visual content may be reduced/dimmed (e.g., a screen, room lighting, etc.), and other properties of the screen/light can also be changed to facilitate sleep (e.g., adjusting the color of ambient lighting).

Reducing the loudness (dB level) of music, voice instructions (or changing the voice to a hushed whisper/murmur), or sound accompanying subject entertainment output by the subject entertainment and guidance system. Alternatively, the sound may be completely turned off. Parameters of the sound may also be altered, like slightly reducing the speed of the sounds/movies to have a more calming effect.

Alerting staff to the fact that the subject is sleeping.

Switch control protocols of apparatus used in the medical examination (e.g., switch to "ComforTone" MRI protocols), or adapting parameters of operation of the apparatus to reduce noise level (e.g. by decreasing gradient strengths and slopes in MRI). (iii) Facilitating wakefulness. When it is required for the subject to be awake due to actions to be taken for the medical examination, or due to subject and clinician preferences, operation parameters of the subject entertainment and guidance system may be automatically adjusted to prompt the subject to be awake/induce an awake state.

In cases in which the medical examination includes breath holds, the subject typically needs to be awake to perform the breath holds. This is also the case for certain fMRI sequences, or other sequences that require active participation of subjects. In another example, the subject may have communicated prior to the exam that they are afraid of losing conscious awareness/control of the unfamiliar situation, and wish to stay awake. Furthermore, there may be instances in which the staff wishes for the subject to remain awake for medical reasons. That is, there may be medical reasons or staff concerns regarding subject health, and so it may be desirable to keep the subject awake and responsive to track such concerns. For example, if the subject is likely to lose consciousness, staff may want them to stay awake so that it is easier to monitor that they are okay.

In addition, it may be beneficial to wake a subject when they are not sleeping calmly, start experiencing apnoea (which may result in them gasping for air). Furthermore, it may be beneficial to keep a subject awake when they need to be awake in the near future. That is, not only is the subject's current sleep/wake state compared to the target wake state, but also to predicted future sleep/wake state (e.g., if the subject falling asleep right before a breath hold scan).

Of course, if the subject is asleep, it may be preferable to alter the operating parameters of the subject entertainment and guidance system in a manner that is least likely to startle the subject suddenly awake. That is, embodiments may provide that gentler forms of inducing an awake state are initially attempted to wake the subject, before slowly increasing the severity of the stimulant. For example, a noise or brightness may be gradually increased. If that does not work, a voice prompt may be output.

Nevertheless, when it is determined that the subject needs to be awake, and the subject is asleep or falling asleep, the following may occur:
Content presented by the subject entertainment and guidance system may be altered to be more mentally stimulating (e.g., quizzes, games), brightness of visual content may be increased (e.g., a screen, room lighting, etc.), and other properties of the screen/light can also be changed to mentally stimulate the subject (e.g., adjusting the color of ambient lighting).

Increase the loudness (dB level) of music, voice instructions, automated/computed generated voice instructions, or sound accompanying subject entertainment output by the subject entertainment and guidance system. Prompt the subject entertainment and guidance system to talk to the subject. Relevant acoustic parameters of the sound may also be altered, such as pitch, rhythm, timbre, tone, etc. Overall, this may help to increase the harshness of the sound output, thus keeping the subject awake.

Alter mode of operation of apparatus of the medical examination. For example, a loud or high pitched noise, or a vibration may be generated. Alternatively, a mode of operation may be selected which generated peripheral nerve stimulation in the subject. Specifically, certain MRI sequences have a high likelihood of causing peripheral nerve stimulation, and some MRI sequences create loud and high pitched noises.

To summarize, the subject entertainment and guidance system may have special sets of operating parameters/settings that may facilitate sleep (e.g., dimmed lights, reduced sound levels) or induce wakefulness (e.g., brighter lights). Based on the medical examination timetable (e.g., whether participation is required by the subject), user preferences and clinician preferences, the target sleep/wake state for a patient can be determined at any point in time. Based on sensor data, it may be determined whether the subject is awake, asleep, or starting to fall asleep (i.e., the current sleep status of the subject can be determined). If the target sleep state is not the same as the current sleep state, the settings of the subject entertainment and guidance system are adjusted in a way that manages the subject toward the target sleep state. Likewise, if a current sleep state is not in line with a sleep state in the near future, settings can be adjusted in such a way that the subject may be managed from the current sleep state, towards the sleep state that is desired in the near future. If the target sleep state does not differ from the current sleep state (or there is no particular target sleep state at present or in the near future), settings may be altered in line with the current sleep state of the subject.

Of course, the above is meant by way of example only, and other means and methods of altering operating parameters of the subject entertainment and guidance system, and additionally the apparatus of the medical examination would be readily apparent to the skilled person.

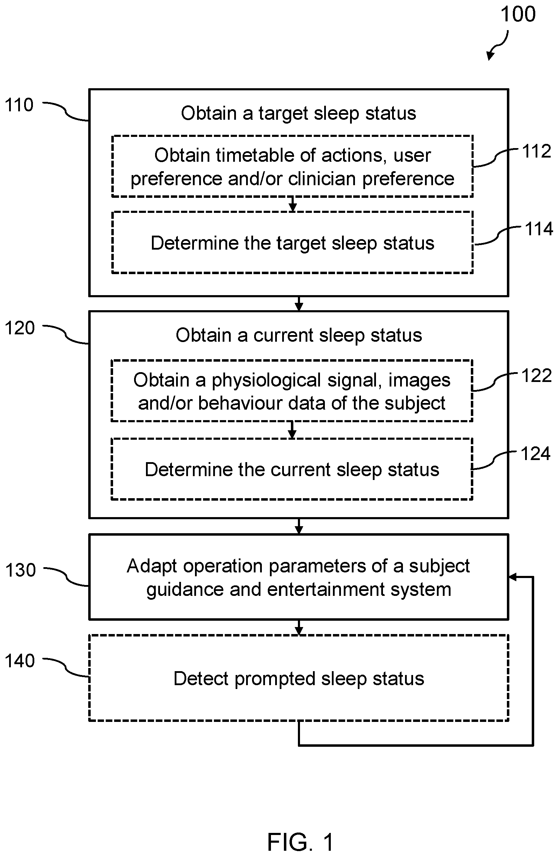

Referring to Fig. 1, a flowchart of a method for managing a subject's sleep status during a medical procedure/examination is depicted. That is, there is provided a method whereby a sleep status of the subject may be adjusted/induced in certain circumstances, and the sleep status of the subject may be facilitated/maintained in other circumstances. The medical procedure may be any medical procedure in which the subject may fall asleep. Accordingly, the depicted method may be particularly suitable for medical procedures where the user is required to remain still for a long period of time, such as a CT or MRI scan.

Generally, when referring to the subject's sleep status, we refer to whether a subject is asleep or awake. Nevertheless, embodiments are not limited hereto. In some embodiments, the sleep status may further describe a trajectory of the subject sleep status (e.g., in the process of waking up, in the process of falling asleep), and optionally a speed of the trajectory (e.g., slowly waking up, rapidly falling asleep). In particular embodiments, the sleep status may also be described in one or more sleep stages. For example, the sleep stage described by the sleep status may be one of wake, non-REM sleep (e.g., N1, N2, N3), and REM. It will be appreciated that, whilst the sleep stage and trajectory is more difficult to detect and control than a simple binary sleep or wake status, this may enable better management of the subject sleep status. That is, subject entertainment and guidance system settings may be more appropriately controlled.

The method begins at step 110, where a target sleep status of the subject is obtained. The target sleep status indicates a desired/preferred status for the subject to experiencing currently and/or in the near feature. This target sleep status may vary throughout the medical examination, or may be a single status throughout the examination. The target sleep status may be obtained from a database, or provided by a clinician, for example.

In some cases, the target sleep status may be based on a timetable of actions that are expected to occur during the medical examination. For instance, the timetable may indicate when the subject is expected to be awake for specific parts of the examination, such as when the subject is expected to follow instructions or participate in an activity. The timetable of actions may therefore vary based on the medical procedure being actioned.

Thus, the timetable of actions may first be obtained in step 112 (e.g., from a database, or provided by a clinician), and then processed in step 114 to determine the target sleep status of the subject. This may be as simple as reading from the examination timetable when the subject requires being awake. Nevertheless, in other cases, ideal sleep stages during each part of the medical examination may be determined as the target sleep status.

In additional and/or alternative embodiments, the target sleep status may be based on a user preference or a clinician preference. For example, a user may prefer to sleep during a lengthy procedure, while a clinician may prefer the subject to be awake during most of the examination. Accordingly, these preferences may be accounted for when determining the target sleep status.

Thus, the user and/or clinician preferences may first be obtained in step 112 (e.g., from a database, or provided by a clinician), and then processed in step 114 to determine the target sleep status of the subject.

The method then proceeds to step 120, where a current sleep status of the subject is detected.

This may involve collecting physiological signals, images, and/or behavior data of the subject in step 122. For instance, physiological signals such as heart rate, breathing rate, and other parameters may be monitored to determine whether the subject is awake or asleep. Images of the subject, such as images of the subject's face, may also be analyzed to detect signs of sleep, such as closed eyes. Behavior data, such as the subject's responses to instructions or participation in activities, may also be used to determine the subject's sleep status. From this data the current sleep status may be determined in step 124. Of course, combinations of the above data may be used to increase a confidence of current sleep status determination. Other means for detecting the current sleep status may also be considered, such as an input from a clinician.

Next, at block 130, one or more operation parameters of a subject guidance and entertainment system are adapted based on the current sleep status and the target sleep status. That is, settings of the subject guidance and entertainment system are altered so as to accommodate for a difference between the current sleep status and the target sleep status.

For example, if the current sleep status indicates that the subject is asleep, but the target sleep status indicates that the subject is expected to be awake for a part of the examination, the operation parameters of the subject guidance and entertainment system may be adjusted to prompt wakefulness. This could involve increasing the brightness of visual content, increasing the volume of auditory content, or starting mentally engaging content to keep the subject awake.

Equally, if the current sleep status indicates that the subject is falling asleep, and the subject may be asleep during the present part of the examination (and in the near future), the operation parameters of the subject guidance and entertainment system may be adjusted to facilitate sleep. This could involve decreasing the brightness of visual content, decreasing the volume of auditory content, or pausing visual and/or auditory content.

Step 130 may involve gradually changing the operation parameters from a current set to a target set. This gradual change may be more comfortable for the subject and may reduce the likelihood of the subject being startled awake, or being frustrated by changes when awake. For example, the brightness of visual content or the volume of auditory content may be gradually increased or decreased to facilitate the transition from sleep to wakefulness or vice versa.

To be clear, the subject guidance and entertainment system may be any system adapted to provide an auditory and/or visual output to the subject during a medical examination/procedure for entertainment purposes (i.e., to keep the subject engaged for a long time) and/or to prompt the subject to perform certain actions.

If the subject guidance and entertainment system comprises a display for observation by the subject during the medical examination, adapting the one or more operation parameters of the subject guidance and entertainment system may comprise adjusting a brightness level of the display, adjusting a color setting of the display, pausing or playing content presented by the display, or adjusting content presented by the display. Of course, the subject guidance and entertainment system may comprise an alternative means for visual output, and thus may have different operation parameters for adjustment. That is, the display may be any device suitable for presenting visual information to the subject. Therefore, the display may encompass, for example, a traditional screen as well as video/virtual reality goggles, a projector, etc.

If the subject guidance and entertainment system comprises a speaker for producing sound audible to the subject during the medical examination, adapting the one or more operation parameters of the subject guidance and entertainment system may comprise adjusting a volume of sound produced by the speaker, adjusting parameters of the sound produced by the speaker, pausing or playing sound produced by the speaker, or adjusting content of the sound produced by the speaker.

In further embodiments, one or more operation parameters of an apparatus for performing the medical examination may be adapted based on the current sleep status and the target sleep status. Thus, similar to the subject guidance and entertainment system, settings of the apparatus itself may be changed to induce or maintain a sleep status of the subject. Indeed, the apparatus may produce sounds and vibrations, as well as cause other effects, which may impact the likelihood of the subject being awake or asleep.

The operation parameters of the subject guidance and entertainment system may be further adapted based on one or more user characteristics. For instance, the system may take into account the user's age, health condition, personal preferences, or other relevant characteristics when adjusting the operation parameters. For example, if the user is a child, the system may adjust the brightness of visual content or the volume of auditory content to levels that are suitable for children. If the user has a health condition that affects their sleep, such as insomnia or sleep apnea, the system may adjust the operation parameters in a way that is tailored to the user's specific sleep patterns. If the user has expressed a preference for a particular type of music or visual content, the system may adjust the operation parameters to play the user's preferred content when the user is awake. This personalized adaptation of the operation parameters may enhance the user's comfort and satisfaction during the medical examination, and may also improve the effectiveness of the system in managing the user's sleep state.

In some aspects, when the sleep status describes a sleep stage of the subject (e.g., wake, light sleep, deep sleep) the operation parameters of the subject guidance and entertainment system may be adjusted to facilitate the transition from the deep sleep stage to the light sleep stage. Thus, fine control over the subject's sleep status may be provided. Indeed, it can be useful to have the subject sleeping lightly (e.g., N1 or N2 sleep), but stop patients from entering deeper sleep phases (e.g., N3 or REM). This may be achieved by, for example, adjusting lighting levels to a point at which the subject does not wake up, but is unlikely to enter deep sleep stages.

More generally, the method may also involve predicting a future sleep status of the subject based on the current sleep status of the subject. This prediction may be used to further adapt the operation parameters of the subject guidance and entertainment system. For example, if the current sleep status indicates that the subject is falling asleep, but the future sleep status predicts that the subject will be awake in the near future, the operation parameters of the subject guidance and entertainment system may be adjusted to facilitate wakefulness.

Finally, in optional step 140, a prompted sleep status of the subject is detected, which may result from the adjustments made to the subject guidance and entertainment system. For instance, if the operation parameters were adjusted to facilitate wakefulness, the prompted sleep status may indicate whether the subject has woken up as a result of these adjustments. If the prompted sleep status indicates that the subject is still asleep, further adjustments may be made to the operation parameters of the guidance and entertainment system to facilitate wakefulness.

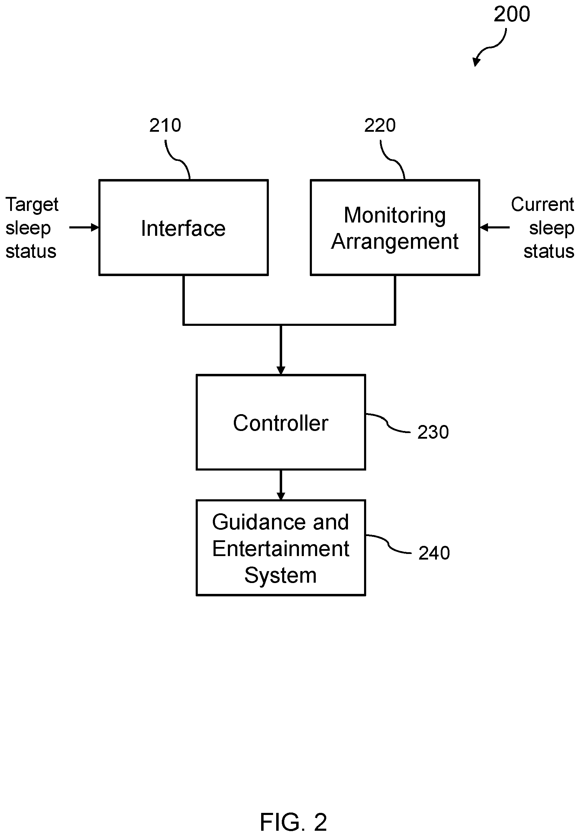

Referring now to Fig. 2, a block diagram of a system for facilitating a subject's sleep/wake state during a medical examination is depicted. The system may include an interface 210, a controller 230, a monitoring Arrangement 220, and a subject guidance and entertainment system 240.

The interface 210 may be configured to receive the target sleep status of the subject. As described above, this target sleep status may be based on a timetable of actions, user preferences, and/or clinician preferences, as previously described.

The controller 230 may be communicatively connected to the interface 210 and the monitoring arrangement 220. The monitoring arrangement 220 may detect the current sleep status of the subject, for example based on various sensors as described herein, and well understood by the skilled person. This could involve using various sensors to monitor physiological signals, images, and/or behavior data of the subject. For instance, physiological signals such as heart rate, breathing rate, and other parameters may be monitored to determine whether the subject is awake or asleep. Images of the subject, such as images of the subject's face, may also be analyzed to detect signs of sleep, such as closed eyes. Behavior data, such as the subject's responses to instructions or participation in activities, may also be used to determine the subject's sleep status. The monitoring arrangement 220 may provide the current sleep status of the subject to the controller 230.

The controller 230 may process this information and adjust the operation parameters of the subject guidance and entertainment system 240 accordingly to manage the subject's sleep/wake state during the medical procedure. The operation parameters of the guidance and entertainment system 240 are adapted based on the current sleep status and the target sleep status.

For instance, if the current sleep status indicates that the subject is asleep, but the target sleep status indicates that the subject is expected to be awake for a part of the examination, the operation parameters of the subject guidance and entertainment system 240 may be adjusted to facilitate wakefulness. This could involve adjusting a brightness level of a display, adjusting a colour setting of the display, pausing or playing content presented by the display, or adjusting content presented by the display.

In other cases, the operation parameters of the subject guidance and entertainment system 240 may be adapted to facilitate sleep. This could involve reducing the brightness of a display, reducing the volume of sound produced by a speaker, pausing content presented by the display, or adjusting content presented by the display to be more soothing or relaxing.

The system may also generate a monitoring signal for indicating the sleep status of the subject to an examiner of the medical examination. This may allow the examiner to manually adjust the operation parameters of the subject guidance and entertainment system or the apparatus for performing the medical examination based on the subject's sleep state.

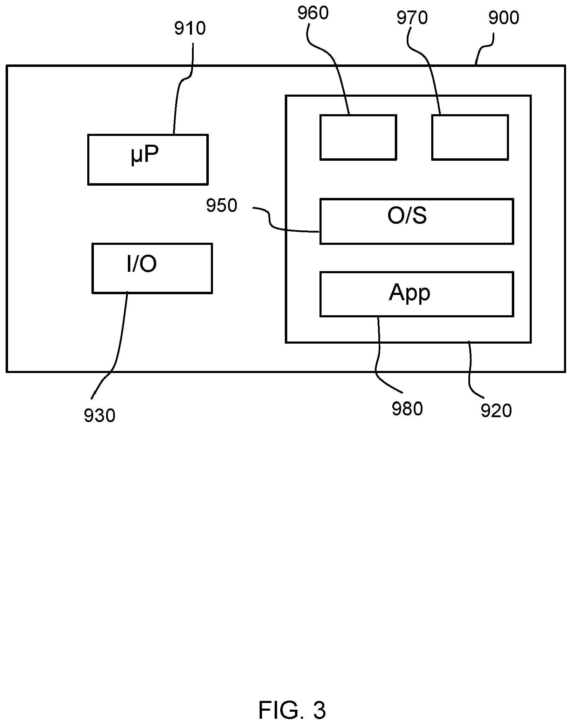

Fig. 3 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A computer-implemented method (100) for managing a sleep state of a subject during a medical examination, the method comprising:
obtaining (110) a target sleep status of the subject;
detecting (120) a current sleep status of the subject;
adapting (130) one or more operation parameters of a subject guidance and entertainment system based on the current sleep status and the target sleep status.

2. The method of claim 1, wherein the target sleep status of the subject is based on a timetable of actions required by the subject in relation to the medical examination, and optionally wherein obtaining the target sleep status comprises:
obtaining (112) the timetable of actions; and
processing (114) the medical examination timetable to determine the target sleep status of the subject.

3. The method of claim 1 or 2, wherein the target sleep status is based on a user preference or a clinician preference, and optionally wherein obtaining the target sleep status comprises:
obtaining (112) the user preference and/or the clinician preference; and
determining (114) the target sleep status of the subject based on the user preference and/or the clinician preference.

4. The method of any of claims 1-3, further comprising a subsequent step of:
detecting (140) a prompted sleep status of the subject responsive to the adaptation of the one or more operation parameters of the subject guidance and entertainment system; and
further adapting one or more operation parameters of the subject guidance and entertainment system based on the prompted sleep status and the target sleep status.

5. The method of any of claims 1-4, further comprising:
predicting a future sleep status of the subject based on the current sleep status of the subject, and optionally a previous sleep status of the subject, and
wherein adapting (130) the one or more operation parameters is further based on the predicted future sleep status.

6. The method of any of claims 1-5, wherein adapting (130) the one or more operation parameters of a subject guidance and entertainment system comprises gradually changing the operation parameters from a current set of operation parameters to a target set of operation parameters, the target operation parameter based on the current sleep status and the target sleep status.

7. The method of any of claims 1-6, wherein detecting (120) the current sleep status of the subject comprises:
obtaining (122) at least one of a physiological signal of the subject during the medical examination, images of the subject during the medical examination, behaviour data describing actions performed by the subject during the medical examination; and
determining (124) the current sleep status of the subject based on the physiological signal, images, and/or behaviour data of the subject.

8. The method of any of claims 1-7, wherein the subject guidance and entertainment system comprises a display for observation by the subject during the medical examination, and wherein adapting (130) the one or more operation parameters of the subject guidance and entertainment system comprises adjusting a brightness level of the display, adjusting a colour setting of the display, pausing or playing content presented by the display, or adjusting content presented by the display.

9. The method of any of claims 1-8, wherein the subject guidance and entertainment system comprises a speaker for producing sound audible to the subject during the medical examination, and wherein adapting (130) the one or more operation parameters of the subject guidance and entertainment system comprises adjusting a volume of sound produced by the speaker, adjusting parameters of the sound produced by the speaker, pausing or playing sound produced by the speaker, or adjusting content of the sound produced by the speaker.

10. The method of any of claims 1-9, further comprising adapting one or more operation parameters of an apparatus for performing the medical examination based on the current sleep status and the target sleep status.

11. The method of any of claims 1-10, further comprising generating a monitoring signal for indicating the sleep status of the subject to an examiner of the medical examination.

12. The method of claim 1, wherein the target sleep status describes a target sleep stage of the subject, and the current sleep status of the subject describes a sleep stage that the subject is currently experiencing, and optionally wherein the sleep stage of the subject includes at least one of wake, N1, N2, N3, and REM.

13. The method of any of claims 1-12, wherein adapting (130) one or more operation parameters of a subject guidance and entertainment system is further based on one or more user characteristics.

14. A computer program comprising computer program code means adapted, when said computer program is run on a computer, to implement the method of any of claims 1 to 13.

15. A system (200) for managing a sleep state of a subject during a medical examination, the system comprising:
an interface (210) configured to obtain a target sleep status of the subject;
a monitoring arrangement (220) configured to detect a current sleep status of the subject; and
a controller (230) configured to adapt one or more operation parameters of a subject guidance and entertainment system (240) based on the current sleep status and the target sleep status.
